(19) Europäisches Patentamt
European Patent Office
Office européen des brevets

(11) EP 2 773 348 B1

(12) **EUROPEAN PATENT SPECIFICATION**

(45) Date of publication and mention of the grant of the patent:
**21.02.2018 Bulletin 2018/08**

(21) Application number: **12780735.2**

(22) Date of filing: **31.10.2012**

(51) Int Cl.:
***A61K 31/4439*** *(2006.01)* ***A61K 9/28*** *(2006.01)*
***A61K 9/50*** *(2006.01)* ***A61P 1/04*** *(2006.01)*

(86) International application number:
**PCT/EP2012/071553**

(87) International publication number:
**WO 2013/064535 (10.05.2013 Gazette 2013/19)**

(54) **PHARMACEUTICAL COMPOSITION OF OMEPRAZOLE**

PHARMAZEUTISCHE OMEPRAZOLZUSAMMENSETZUNG

COMPOSITIONS PHARMACEUTIQUES D'OMÉPRAZOLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.11.2011 EP 11382336**

(43) Date of publication of application:
**10.09.2014 Bulletin 2014/37**

(73) Proprietor: **Laboratorios del Dr. Esteve, S.A.**
**08041 Barcelona (ES)**

(72) Inventors:
- **SANGRÀ PEREZ, Jaume**
  **E-08860 Castelldefels (Barcelona) (ES)**
- **ALCOCER ARANZANA, Cristina**
  **E-08940 Cornellá de Llobregat (Barcelona) (ES)**

(74) Representative: **ABG Patentes, S.L.**
**Avenida de Burgos, 16D**
**Edificio Euromor**
**28036 Madrid (ES)**

(56) References cited:

| | |
|---|---|
| **EP-A- 1 108 425** | **EP-A1- 1 552 833** |
| **EP-A2- 1 086 694** | **WO-A1-96/01623** |
| **WO-A1-96/01624** | **WO-A1-2004/098573** |
| **WO-A2-2004/016242** | **WO-A2-2004/096218** |
| **WO-A2-2011/054930** | **WO-A2-2012/092486** |
| **US-A- 5 385 739** | **US-A- 5 626 875** |

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 773 348 B1

## Description

### FIELD OF THE INVENTION

The present invention refers to an oral solid pharmaceutical composition comprising omeprazole pellets.

### BACKGROUND

Omeprazole or 5-methoxy-2-(4-methoxy-3,5-dimethylpyridin-2-ylmethylsulfinyl)-1H-benzimidazole (marketed in Europe under the trademark Losec®) is a substituted benzimidazole that inhibits gastric acid secretion. The empirical formula of omeprazole is $C_{17}H_{19}N_3O_3S$ and the compound has a molecular weight of 345.41. The structural formula of omeprazole is (I):

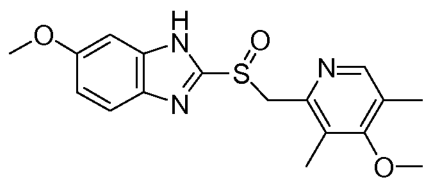

(I)

Omeprazole is a white to off-white crystalline powder that melts with decomposition at about 155°C. It is a weak base, freely soluble in ethanol and methanol, and slightly soluble in acetone and isopropanol and very slightly soluble in water. The stability of omeprazole is a function of pH; it is rapidly degraded in acid media, but has acceptable stability under alkaline conditions.

Omeprazole belongs to a class of antisecretory compounds called proton pump inhibitors ("PPIs") that are prescribed, *inter alia*, for short-term treatment of active duodenal ulcers, gastric ulcers, gastroesophageal reflux disease (GERD), severe erosive esophagitis, poorly responsive systematic GERD, and pathological hypersecretory conditions such as Zollinger Ellison syndrome.

PPIs are, however, susceptible to degradation/transformation in acidic and neutral media. The degradation is catalyzed by acidic compounds and is stabilized in mixtures with alkaline compounds. The stability of the active substances is also affected by moisture, heat, organic solvents and to some degree by light.

With respect to the stability properties of the active substances, it is known that an oral dosage form should be protected from contact with the acidic gastric juice or comprise suitable components to neutralise the acidic gastric juice so that the active substance can be transferred in intact form to that part of the gastrointestinal tract where pH is near neutral and where rapid absorption can occur.

Accordingly, in a pharmaceutical oral dosage form, omeprazole is best protected from contact with acidic gastric juice when said dosage form contains an enteric coating layer. For oral administration, commonly used solid dosage forms are capsules and tablets comprising a multitude of enteric coated pellets of the active ingredient. For example, EP 1 086 694 A2 discloses a solid pharmaceutical formulation of modified release comprising a number of pellets that contain an acid labile benzimidazole compound as an active ingredient, and one or more intermediate layers that comprise, at least, a system of sustained release, and an external enteric coating. The weight percentage of benzimidazole compound (or specifically omeprazole) employed in the oral solid pharmaceutical compositions of EP 1 086 694 A2 is at most equal to about 6 to 9% by weight of the pellet.

WO 2011/054930 discloses pharmaceutical oral solid dosage forms comprising: (i) a core comprising a benzimidazole; (ii) a separating layer comprising a water soluble polymer and glyceryl monostearate; and (iii) an enteric coating.

WO 96/01623 discloses an oral pharmaceutical multiple unit tableted dosage form comprising tablet excipients and individually enteric coating layered units of a core material containing omeprazole or a salt thereof, covered with one or more layer(s).

EP 1552833 discloses solid dosage forms for medicinal use containing an amorphous benzimidazole compound, such as omeprazole, where said solid dosage forms can be granules, capsules, tablets and effervescent dosage forms. The disclosed dosage forms have an intermediate coating formed on a layer containing the amorphous benzimidazole compound, and a controlled release coating layer and/or enteric coating formed on said intermediate coating.

WO 2012/092486 relates to modified release benzimidazole formulations comprising one or more particles containing omeprazole or a salt thereof and having a drug release modifying coating being soluble at pH values about 5 to about 7.5; and one or more particles containing omeprazole or a salt thereof having a polymer coating soluble at

pH values about 5 to about 6.

EP 1 108 425 relates to oral multi-unitary pharmaceutical preparations containing substituted benzimidazoles or their pharmaceutically acceptable salts. Such pharmaceutical preparations are stable pellet preparations comprising a quantity of active ingredient of between 1 and 50 mg, an inert core of spherical symmetry with a diameter of 600 - 1000 $\mu$m, constituted by inert excipients, coated with an active layer containing at least one substituted benzimidazole in the micronized form and various pharmaceutically acceptable inert excipients, mixed in suitable proportions in order to allow the disaggregation of the formulations and dissolution of the active ingredient(s) in an appropriate manner, coated in turn with an insulating layer of a strictly polymeric nature, soluble in water, free from alkaline and/or alkaline-earthy metallic salts, of a minimum thickness of 15 $\mu$m, this layer being coated lastly with a gastroresistant or enteric layer of a minimum thickness of 30 $\mu$m.

US 5 385 739 relates to a stable formulation of omeprazole microgranules containing a neutral core consisting of sugar and starch, characterized in that it contains an active layer consisting of a dilution of omeprazole in mannitol in substantially equal amounts.

WO 2004/016242 relates to a process for manufacture of a stable, oral, multiple unit pharmaceutical composition containing upto about 40%w/w of benzimidazole, wherein the process involves sequential deposition of a) alkaline material layer on non-pariel seeds to obtain treated non-pariel seeds, b) drug layer to obtain drug pellets, c) sealant polymer layer to obtain sealed pellets, and d) enteric polymer layer to obtain enteric coated pellets, and wherein the enteric coated pellets have minimum acid degradation and buffer release of not less than 85% after 45 minutes and are capable of being filled into size 5 to size 0 capsule, the pharmaceutical composition being exempt of surfactants in contact with benzimidazole, disintegrating agents and fillers.

WO 2004/096218 discloses pellet formulations of acid-labile antiulcer benzimidazole compounds comprising inert granules of sugar/starch which are: initially coated with a non-alkaline active layer having the benzimidazole compound (omeprazole, lansoprazole, pantoprazole, rabeprazole, etc.), sodium and/potassium salts of acids of formula R-O-$SO_3H$ wherein R is an alkyl radical of a ($C_6$-$C_{20}$)-fatty acid, non-alkaline pharmaceutically acceptable disintegrants and non-alkaline pharmaceutically acceptable binders; secondly coated with a non-alkaline barrier layer comprising one or more pharmaceutically acceptable coating excipients; and finally coated with an enteric layer. The active layer is characterized by having a substantial amount of ($C_6$-$C_{20}$)-fatty acids and a substantial amount of sodium and/or potassium salts of ($C_6$-$C_{20}$)-fatty acids, these two amounts being in a molar ratio [acids]: [salts] between 1: 4 and 4: 1.

In spite of this background, it would be highly desirable to provide new omeprazole containing dosage forms in which the omeprazole is present in a high concentration, in particular, oral solid pharmaceutical compositions comprising concentrate omeprazole pellets. Such a kind of pharmaceutical composition is advantageous since it would require a lower number of pellets and/or smaller pellets, thus allowing a decrease in the quantity of excipients as well as the formulation in smaller dosage forms (such as capsules of limited size). Preferably all this would result in both economic (cheaper cost of formulation, packaging, storage, transportation, etc.) and operational advantages (more flexibility in formulating or facilitating its formulation, improvement of the production capacity). Also, such pharmaceutical composition will have advantage with patients with swallowing difficulties as elderly and children, enhancing the treatment adherence or compliance for these patients. Of course, such new forms should offer suitable gastroresistance as well.

Therefore, there is still a need for further dosage forms of omeprazole showing a high concentration of this active ingredient while exhibiting at the same time good physicochemical stability.

## BRIEF DESCRIPTION OF THE INVENTION

The inventors of the present invention have surprisingly developed concentrate omeprazole pellets able to afford an oral solid pharmaceutical composition which is chemically and physically stable, has a good *in vitro* dissolution rate and needs a capsule size smaller than the reference product (Losec®).

In particular, one aspect of the present invention relates to a pellet (also referred herein simply as pellet of the invention) which comprises:

a) a core comprising omeprazole or a pharmaceutically acceptable salt thereof;

b) at least an intermediate layer; and

c) an enteric coating layer comprising an enteric polymer;

and characterized in that the weight percentage of the enteric coating layer is between 5% (w/w) and 40% (w/w) with respect to the total weight of the pellet and wherein the core (a) comprises an inert nucleus having a diameter average size lying between about 0.3 mm and 0.9 mm.

In a more particular embodiment, the present invention relates to a pellet which comprises:

a) a core comprising omeprazole or a pharmaceutically acceptable salt thereof;

b) at least an intermediate layer; and

c) an enteric coating layer comprising an enteric polymer;

and characterized in that the weight percentage of the enteric coating layer is between 20% (w/w) and 40% (w/w) with respect to the total weight of the pellet and wherein the core (a) comprises an inert nucleus having a diameter average size lying between about 0.3 mm and 0.9 mm.

Advantageously, the pellet of the invention allows preparing pharmaceutical compositions in which the weight percentage of omeprazole is between 10% (w/w) and 25% (w/w) with respect to the total weight of the pellets comprised in the pharmaceutical composition.

In a further aspect, the present invention relates to a process for manufacturing a pellet which comprises:

a) preparing a core comprising omeprazole or a pharmaceutically acceptable salt thereof;

b) coating said core with at least an intermediate layer; and

c) coating said intermediate layer(s) with an enteric coating layer comprising an enteric polymer, in which the weight percentage of the enteric coating layer is between 5% (w/w) and 40% (w/w) with respect to the total weight of the pellet; and wherein the core comprises an inert nucleus having a diameter average size lying between about 0.3 mm and 0.9 mm.

According to another aspect, the invention relates to an oral solid pharmaceutical composition (also referred herein simply as composition of the invention) comprising the omeprazole pellets as defined herein. Preferably, the pellets are filled into a capsule, in particular a gelatine capsule.

In a further aspect, the present invention relates to the pharmaceutical composition as defined herein for use in the treatment and/or prophylaxis of a gastrointestinal disorder.

In a further aspect, the present invention relates to the use of the pharmaceutical composition as defined herein for the preparation of a medicament for the treatment and/or prophylaxis of a gastrointestinal disorder.

Another aspect of the present invention refers to a method for the treatment and/or prophylaxis of a gastrointestinal disorder, the method comprising administering to the subject in need of such a treatment or prophylaxis a therapeutically effective amount of the pharmaceutical composition described herein.

In one embodiment, the pharmaceutical composition of the invention is indicated for treatment and/or prophylaxis of ulcers of the stomach and duodenum, and NSAID-induced ulcers; gastroesophageal reflux disease (GERD) (also known as acid reflux disease); severe erosive esophagitis; poorly responsive systematic GERD; pathological hypersecretory conditions such as Zollinger-Ellison Syndrome; and adjunctive treatment of *Helicobacter pylori* infection, alongside antibiotics; or combinations of any of the above disorders.

These aspects and preferred embodiments thereof are additionally also defined hereinafter in the detailed description, as well as in the claims.

## DETAILED DESCRIPTION OF THE INVENTION

The technical problem of the present invention is to provide an alternative oral solid pharmaceutical composition comprising concentrate omeprazole pellets that is chemically and physically stable, has a good in vitro dissolution rate which exhibits good bioavailability and results in bioequivalence between the test and reference products.

The inventors have surprisingly found that an oral solid pharmaceutical composition comprising concentrate omeprazole pellets and an inert nucleus having a diameter average size lying between about 0.3 mm and 0.9 mm provides a concentrated oral solid pharmaceutical composition of omeprazole pellets so that a lower number of pellets and/or smaller pellets are required, thus allowing the formulation in smaller dosage forms. In addition to an optimum dissolution profile, the formulation of the invention presents the advantage of exhibiting excellent storage stability.

The inventors have found that when decreasing size and quantity of pellets to be used and increasing quantity of omeprazole, the enteric coat has to be modulated to keep protective properties and dissolution profile. According to the present invention, the amount of enteric coating is modified and it is present in the pellet of the invention in an amount lying between about 5% and 40%, preferably between about 20% and 40% by weight of the pellet.

Moreover, the oral pharmaceutical composition of the present invention presents the advantage that it is cost effective since requires a lower number of pellets and/or smaller pellets, thus allowing a decrease in the quantity of excipients as well as the formulation in smaller dosage forms (such as capsules of limited size) and also a cheaper

packaging cost.

In the context of the present invention, the following terms have the meaning detailed below.

By "pharmaceutically acceptable" such as in the recitation of a "pharmaceutically acceptable salt" or a "pharmaceutically acceptable excipient" is meant herein a material that is not biologically or otherwise undesirable, i.e., the material may be incorporated into a pharmaceutical composition administered to a patient without causing any undesirable biological effects or interacting in a deleterious manner with any of the other components of the composition in which it is contained.

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. Examples of pharmaceutically acceptable salts of omeprazole include a salt with an inorganic base, a salt with an organic base, a salt with a basic amino acid and the like. Preferred examples of the salt with an inorganic base include alkali metal salts such as a sodium salt and a potassium salt; alkaline earth metal salts such as a calcium salt and a magnesium salt; an ammonium salt and the like. A more preferred example is in particular the magnesium salt.

By the term "equivalence" or "bioequivalence" it is understood the following: two medicinal products containing the same active substance are considered bioequivalent if they are pharmaceutically equivalent or pharmaceutical alternatives and their bioavailabilities (rate and extent) after administration in the same molar dose lie within acceptable predefined limits (with a 90 percent confidence interval of ratio of the geometric mean between the test and the reference within 80.00 and 125.00 percent). These limits are set to ensure comparable *in vivo* performance, i.e. similarity in terms of safety and efficacy. See Guideline on the Investigation of Bioequivalence. European Medicines Agency. Doc. Ref.: CPMP/QWP/EWP/1401/98 Rev. 1.

The term "smaller dosage form" in the context of the invention means a capsule size smaller than the one that the skilled person in the art would have obtained applying the teachings of the state of art in order to get an oral solid pharmaceutical composition having a higher concentration of omeprazole. The present invention allows a formulation in a smaller capsule size; in particular, the present invention allows a capsule size smaller than the reference product in the market (Losec®).

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Unless otherwise stated, all amounts are expressed herein as percentage by weight. The weight of the pellets must be understood as dry weight.

Pellet - Pharmaceutical composition

The pellets of the invention contain omeprazole or a pharmaceutically acceptable salt thereof as active ingredient and advantageously are manufactured in multilayer form having a core-sheath structure, as known from the state of the art. Thus, the pellets proposed herein, in essence, comprise: a) a core, b) one or more intermediate layers disposed over said core, and c) an enteric coating layer disposed over said intermediate layers.

Preferentially, the nucleus of the pellets may be made by an inert bead which is covered with a layer comprising the active ingredient and optionally pharmaceutically acceptable excipients. The nucleus is inert with regard both to omeprazole and to the other excipients in the pellet, and with regard to the patient who will ingest the pellet. Such inert bead is conventionally used in pharmaceutical techniques. The bead may be prepared from materials such as, e. g. starch, sucrose, microcrystalline cellulose, and the like. As noted above, the present invention has as an objective to obtain small dosage forms of omeprazole such as capsules. In this context, with the objective of reducing the size of the capsule, the amount and size of inert beads has to be determined in order to estimate the total surface on which omeprazole layer is incorporated so that after adding the sealing layer and the enteric layer the obtained pellets have the optimum size for a convenient capsule dosage. An average size of diameter lying between about 0.3 mm and 0.9 mm, preferably between about 0.5 mm and 0.9 mm, more preferably between about 0.7 mm and 0.9 mm, even more preferably about 0.8 mm, has been found to be suitable for the inert beads according to the present invention. In a particular embodiment, the inert bead (or nucleus) is present in the pellet of the invention in an amount lying between about 20% and 50%, preferably between 20% and 45%, by weight of the pellet. In some preferred embodiments, the inert bead is present in an amount lying between about 20% and 35%, preferably about 31%, by weight of the pellet. In other preferred embodiments, the inert bead is present in an amount lying between about 35% and 45%, preferably

about 40%, by weight of the pellet.

The amount of active ingredient (i.e. omeprazole or a salt thereof) may vary for example from 10% to 25% with respect to the weight of the pellet. In particular, amounts of about 12% to 17% (w/w) have been found to be preferred. The layer containing the active ingredient (herein referred to as active layer; film coating 1 in the examples) may include excipients commonly used in pharmaceutical formulations that do not interact adversely with omeprazole and its salts. In a preferred embodiment, the core of the pellets comprises an inert bead and said inert bead is covered by an active layer comprising omeprazole, or a pharmaceutically acceptable salt, and pharmaceutically acceptable excipients, such as at least a binder. Examples of binders include, but are not limited to, hydroxypropylcellulose (or HPC), hydroxypropylmethylcellulose (or HPMC or hypromellose), hydroxyethyl cellulose, sugars (such as sucrose, glucose and dextrose), or a combination thereof. In addition to the foregoing, the active layer can further contain other excipients such as disintegrants, anti-tacking agents, stabilizing agents and the like. The following are examples of useful disintegrants: starches such as corn or potato starch, modified starches (such as sodium starch glycolate) and partially pregelatinized starches (such as Starch 1500); polyvinylpyrrolidones, including modified polyvinylpyrrolidones (such as crospovidone, polymerized under conditions that promote crosslinking); celluloses such as microcrystalline cellulose, modified celluloses (such as low substituted hydroxypropyl cellulose, croscarmellose sodium and calcium carboxymethyl cellulose). As stabilizing agents, alkaline compounds such as disodium phosphate dihydrate, magnesium carbonate or sodium carbonate could be used. Example of anti-tacking agent is talc.

In a more preferred embodiment, the pellet of the invention comprises a core comprising omeprazole or a pharmaceutically acceptable salt thereof, a binder, a stabilizing agent and an anti-tacking agent.

In a still more preferred embodiment, the pellet of the invention comprises a core comprising omeprazole or a pharmaceutically acceptable salt thereof, hydroxypropylmethylcellulose, disodium phosphate dihydrate and talc.

The pellets are individually enteric coated by one or more layers. Before applying enteric coating layer(s), said pellets are covered with one or, optionally, more intermediate layers comprising pharmaceutical excipients. This/these intermediate layer(s) separate(s) the core comprising the active layer of omeprazole from the outer layer(s) being enteric coating layer(s).

The intermediate layer(s) can be applied to the active layer by coating or layering procedures in suitable equipments such as coating pan, coating granulator or in fluidized bed apparatus using water and/or organic solvents for the coating process. As an alternative, the separating layer(s) can be applied to the active layer by using powder coating technique. The materials for separating layers are pharmaceutically acceptable excipients such as, for instance, binders (e.g. hydroxypropylcellulose or HPC, hydroxypropylmethylcellulose or HPMC or hypromellose, hydroxyethyl cellulose), sugars (such as sucrose, glucose and dextrose), disintegrants (e.g. corn or potato starch, sodium starch glycolate, polyvinylpyrrolidones, including modified polyvinylpyrrolidones such as crospovidone, polymerized under conditions that promote crosslinking; celluloses such as microcrystalline cellulose, modified celluloses such as low substituted hydroxypropyl cellulose, croscarmellose sodium and calcium carboxymethyl cellulose), surfactants (e.g. polysorbates, sodium laurylsulfate), plasticizers (polyethylene glycol, acetylated monoglyceride, triacetin, castor oil and the like), lubricants (e.g. sodium stearylfumarate, magnesium stearate, hydrogenated vegetable oil, stearic acid, calcium stearate, glyceryl behenate, sodium lauryl sulphate), anti-tacking agents (e.g. talc), colorants (e.g. titanium dioxide), used alone or in mixtures.

The intermediate layer(s) may improve the chemical stability of the active ingredient and/or the physical properties of the pharmaceutical composition. According to particular embodiments, the pellets of the invention comprise one of the following intermediate layers or both:

- an intermediate layer comprising at least a binder. Preferably, this kind of intermediate layer comprises hydroxypropylmethylcellulose, talc and titanium dioxide; and/or

- an intermediate layer comprising a non-alkaline polymer insoluble in water such as ethylcellulose (e.g. Surelease E-7-7050). This intermediate layer preferably comprises further an inert polymer, non-alkaline, soluble in water such as hydroxypropylmethylcellulose (also referred to as HPMC or hypromellose) in order to form a system of modified release,

If both aforementioned intermediate layers are present, it is preferred that the intermediate layer comprising a non-alkaline polymer insoluble in water is disposed over (outer) the other intermediate layer. However, the arrangement of the these intermediate layers is not restricted to said disposition and consequently, the present invention also encompasses pellets having the intermediate layer comprising at least a binder disposed over the intermediate layer comprising a non-alkaline polymer insoluble in water.

One or more enteric coating layers are applied onto the active layer covered with intermediate layer(s) by using a suitable coating technique. The enteric coating layer material may be dispersed or dissolved in either water or in suitable organic solvents. As enteric coating layer polymers one or more, separately or in combination, of the following

can be used; e.g. solutions or dispersions of methacrylic acid copolymers, polysorbates, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac, or other suitable enteric coating layer polymer(s). Methacrylate-base coatings are preferred and several useful products are commercially available from Rohm GmbH & Co., Darmstadt, Germany under the trademark EUDRAGIT. EUDRAGIT L30D-55 is especially preferred. EUDRAGIT L30D-55 is an aqueous dispersion of a pH dependent polymer soluble at or above pH 5.5 for targeted delivery in the duodenum. The methacrylic acid copolymer EUDRAGIT L30D-55 is a copolymer of methacrylic acid an ethyl acrylate in a 1:1 ratio and has the formula $(C_5H_2O_2{\cdot}C_4H_6O_2)_X$.

The enteric coating layers may further contain pharmaceutically acceptable plasticizers to obtain the desired mechanical properties, such as flexibility and hardness of the enteric coating layers. Such plasticizers are for instance, but not restricted to, triacetin, citric acid esters, phthalic acid esters, dibutyl sebacate, cetyl alcohol, polyethylene glycols, polysorbates or other plasticizers. Anti-tacking agents, such as for instance magnesium stearate, titanium dioxide, talc and other additives may also be included into the enteric coating layer(s), being titanium dioxide and/or talc preferred.

As noted above, an outstanding problem of PPIs is to avoid the protonic transfer from the external medium at pH acid, to the core that contains the acid labile compound.

Thus, in order to provide a suitable formulation of acid labile compounds, there is an existing need to avoid the proton transfer from the external medium to the core since this migration would affect negatively to the API's stability. In this sense, EP 1086694 A2 discloses benzimidazole pellets having an enteric coating in an amount ranging from 10-15%. However, the inventors have found that when decreasing size and quantity of pellets to be used and increasing quantity of omeprazole, the enteric coat has to be modulated to keep protective properties and dissolution profile. According to the present invention, the amount of enteric coating is modified and it is present in the pellet of the invention in an amount lying between about 5% and 40% by weight of the pellet. In a particular embodiment the lower amount of the enteric coating in the preceding range can be advantageously chosen from 6%, 7%, 8%, 9% or 10%, thus more preferred ranges are selected from between about 6% to 40%, 7% to 40%, 8% to 40%, 9% to 40% or 10% to 40% by weight of the pellet. In particular, amounts of about 20% to 40% (w/w) have been found to be preferred in some embodiments, and more preferably the amount of enteric coating is between about 25% and 35% by weight of the pellet, such as about 31%. In other preferred embodiments the amount of enteric coating is between about 5% and 20% by weight of the pellet and more preferably the amount of enteric coating is between about 7% and 17% by weight of the pellet, such as about 10%, about 13% or about 16%.

In a particular embodiment, the enteric coating comprises (i) an enteric polymer such as a methacrylate copolymer, (ii) a plasticizer such as triethyl citrate and iii) an anti-tacking agent such as talc. In a more preferred embodiment, the pellet of the invention comprises an enteric coating comprising methacrylic acid copolymer (Eudragit L30D-55), triethyl citrate and talc.

In a more preferred embodiment, the pellet of the invention comprises:

a) a core comprising omeprazole or a pharmaceutically acceptable salt thereof, hydroxypropylmethylcellulose, disodium phosphate dihydrate and talc;

b) an intermediate layer comprising hydroxypropylmethylcellulose, talc and titanium dioxide;

c) optionally, another intermediate layer comprising ethylcellulose and hydroxypropylmethylcellulose; and

d) an enteric coating layer comprising methacrylic acid copolymer (e.g. Eudragit L30D-55), triethyl citrate and talc.

Further, the pharmaceutical composition of the invention may be in the form of a compressed dosage form such as a tablet, or alternatively, the pellets may be filled into capsules or sachets. Preferably, the pharmaceutical composition of the invention is provided in form of capsules e.g., soft or hard gelatin and non-gelatin capsules. Non-gelatin capsules are for instance those made up of plant polysaccharides or their derivatives (like carrageenans and modified forms of starch and cellulose).

In a preferred embodiment, the capsule further comprises a lubricant, which is preferably selected from sodium stearylfumarate, magnesium stearate, hydrogenated vegetable oil, stearic acid, calcium stearate, glyceryl behenate, sodium lauryl sulphate and talc, the latter being preferred. The combination of above-mentioned lubricants can also be used. Typically the lubricant(s) is present in less than 1% by weight with respect to the sum of the weights of pellets and lubricant(s).

In additional preferred embodiments, the preferences described above for the components of the pellets are combined. The present invention is also directed to such combinations of preferred components within the pellet.

Process

The process for the manufacture of the pellet represents a further aspect of the invention. The mentioned formulations will be prepared using standard methods such as those described or referred to in the European and US Pharmacopoeias and similar reference texts. The pharmaceutical processes can preferably be completely water-based and there are different descriptions given in the accompanying examples below.

In a particular embodiment, the process for manufacturing the pellet of the invention comprises the steps of:

a) preparing a core comprising omeprazole or a pharmaceutically acceptable salt thereof;

b) coating said core with at least an intermediate layer; and

c) coating said intermediate layer with an enteric coating layer comprising an enteric polymer.

As previously described, the at least an intermediate layer may be selected according to a particular embodiment from an intermediate layer comprising at least a binder, an intermediate layer comprising a non-alkaline polymer insoluble in water and these both layers, in any disposition.

Use of the pharmaceutical composition

The pharmaceutical composition according to the invention is especially advantageous in reducing gastric acid secretion.

In a further aspect, the present invention relates to a method of treating a gastrointestinal disorder. The method involves the step of administering to a patient in need of such a treatment (notably a human) a therapeutically effective amount of the pharmaceutical composition described herein. Gastrointestinal disorders that can be treated using the hereinbefore described method include, but are not limited to, ulcers of the stomach and duodenum, and NSAID-induced ulcers; gastroesophageal reflux disease (GERD) (also known as acid reflux disease); severe erosive esophagitis; poorly responsive systematic GERD; pathological hypersecretory conditions such as Zollinger-Ellison Syndrome; and adjunctive treatment of *Helicobacter pylori* infection, alongside antibiotics; or combinations of any of the above disorders.

In one embodiment, the pharmaceutical composition of the invention is specifically indicated for short-term treatment of active duodenal ulcer, H. pylori eradication to reduce the risk of duodenal ulcer recurrence, maintenance of healed duodenal ulcers, short-term treatment of active benign gastric ulcer, healing of NSAID-associated gastric ulcer, risk reduction of NSAID-associated gastric ulcer, short-term treatment gastroesophageal reflux disease (GERD), maintenance of healing of erosive esophagitis (EE), long-term treatment pathological hypersecretory conditions Including Zollinger-Ellison Syndrome (ZES).

Generally an effective administered amount of omeprazole will depend on the severity of the disorder being treated and the weight of the sufferer. However, the pharmaceutical composition of the invention will typically be administered once or more times a day for example 1, 2, 3 or 4 times daily, with typical total daily doses of omeprazole in the range of from 10 to 200 mg, such as 10, 20 or 40 mg.

The following examples are merely illustrative of certain embodiments of the invention and cannot be considered as restricting it in any way.

**Examples**

The pellets of examples 1-8 were prepared following the procedure described below.

1. FC 1 (Film Coating No. 1) was prepared as follows: the required amount of omeprazole, hypromellose, disodium phosphate dehydrate and talc was dispersed into purified water by constant stirring. The suspension was homogenised and maintained under constant stirring until required.

2. Inert beads were coated by pulverising the suspension FC 1 using in a fluid bed drying granulator. After spraying and before applying the second layer, the spheres obtained were dried.

3. FC 2 (Film Coating No. 2) was prepared by dispersing hypromellose, talc and titanium dioxide into purified water by constant stirring.

4. FC 1 coated beads were further coated by pulverising the suspension FC 2. After spraying and before applying the following layer, the spheres obtained were dried.

5. Optionally, another coating layer (FC 3 or Film Coating No. 3) after FC 2 was prepared using Surelease E-7-7050 (ethylcellulose), hypromellose and water, said FC 3 being subsequently sprayed onto the spheres before applying the Enteric Film Coating.

6. Enteric film coating was prepared by dispersing methacrylic acid copolymer (Eudragit L30D-55), triethylcitrate (E-1505) and talc into purified water by constant stirring. The suspension was homogenised and maintained under constant stirring until required.

7. The pellets of step 4 or 5 were coated by pulverising the suspension Enteric Film Coating using a fluid bed drying granulator.

8. Finally, the pellets were dried and sieved.

**EXAMPLE 1:**

Omeprazole delayed release pellets containing an enteric layer of methacrylic acid copolymer and an intermediate layer. Each film coating (FC) suspension was made and sprayed over the preceding layer in a fluid bed apparatus according with the following components:

| Coat | Component | mg per dose 40mg |
|---|---|---|
| **Nucleus** | Inert beads | 80.00 |
| **FC 1** | Omeprazole<br>Hypromellose<br>Disodium phosphate dihydrate<br>Talc<br>Purified water | 40.00<br>30.57<br>10.00<br>9.57<br>309.51 |
| **FC 2** | Hypromellose<br>Talc<br>Titanium dioxide<br>Purified water | 16.58<br>2.21<br>2.21<br>110.61 |
| **Enteric coat** | Eudragit L30D-55, 30% dry content<br>Triethyl citrate<br>Talc<br>Purified water | 153.71<br>6.94<br>6.94<br>148.18 |
| **Total** | | 251.13 |

**EXAMPLE 2:**

Omeprazole delayed release pellets containing an enteric layer of methacrylic acid copolymer and an intermediate layer. Each film coating (FC) suspension was made and sprayed over the preceding layer in a fluid bed apparatus according with the following components:

| Coat | Component | mg per dose 40mg |
|---|---|---|
| **Nucleus** | Inert beads | 94.00 |
| **FC 1** | Omeprazole<br>Hypromellose<br>Disodium phosphate dihydrate<br>Talc<br>Purified water | 40.00<br>30.56<br>10.00<br>9.57<br>154.75 |

(continued)

| Coat | Component | mg per dose 40mg |
|---|---|---|
| FC 2 | Hypromellose<br>Talc<br>Titanium dioxide<br>Purified water | 17.69<br>2.36<br>2.36<br>117.99 |
| Enteric coat | Eudragit L30D-55, 30% dry content | 247.98 |
| | Triethyl citrate<br>Talc<br>Purified water | 11.20<br>11.20<br>239.07 |
| Total | | 303.33 |

**EXAMPLE 3:**

Omeprazole delayed release pellets containing an enteric layer of methacrylic acid copolymer, an intermediate layer and another coat containing Surelease. Each film coating (FC) suspension was made and sprayed over the preceding layer in a fluid bed apparatus according with the following components:

| Coat | Component | mg per dose 20mg |
|---|---|---|
| Nucleus | Inert beads | 40.00 |
| FC 1 | Omeprazole<br>Hypromellose<br>Disodium phosphate dihydrate<br>Talc<br>Purified water | 20.00<br>15.28<br>5.00<br>4.78<br>154.75 |
| FC 2 | Hypromellose<br>Talc<br>Titanium dioxide<br>Purified water | 8.85<br>1.18<br>1.18<br>58.99 |
| FC 3 | Surelease E-7-7050 (ethylcellulose)<br>Hypromellose<br>Purified water | 26.16<br>5.35<br>93.32 |
| Enteric coat | Eudragit L30D-55, 30% dry content<br>Triethyl citrate<br>Talc<br>Purified water | 74.35<br>3.35<br>3.35<br>72.01 |
| Total | | 137.17 |

**EXAMPLE 4:**

Omeprazole delayed release pellets containing an enteric layer of methacrylic acid copolymer, an intermediate layer and another coat containing Surelease. Each film coating (FC) suspension was made and sprayed over the preceding layer in a fluid bed apparatus according with the following components:

| Coat | Component | mg per dose 20mg |
|---|---|---|
| Nucleus | Inert beads | 40.00 |

(continued)

| Coat | Component | mg per dose 20mg |
|---|---|---|
| **FC 1** | Omeprazole<br>Hypromellose<br>Disodium phosphate dihydrate<br>Talc<br>Purified water | 20.00<br>15.28<br>5.00<br>4.78<br>154.75 |
| **FC 2** | Hypromellose<br>Talc<br>Titanium dioxide<br>Purified water | 8.85<br>1.18<br>1.18<br>58.99 |
| **FC 3** | Surelease E-7-7050 (ethylcellulose)<br>Hypromellose<br>Purified water | 41.80<br>8.55<br>149.12 |
| **Enteric coat** | Eudragit L30D-55, 30% dry content<br>Triethyl citrate<br>Talc<br>Purified water | 128.19<br>5.77<br>5.77<br>124.16 |
| **Total** | | 165.27 |

**EXAMPLE 5:**

Omeprazole delayed release pellets containing an enteric layer of methacrylic acid copolymer and an intermediate layer. Each film coating (FC) suspension was made and sprayed over the preceding layer in a fluid bed apparatus according with the following components:

| Coat | Component | mg per dose 20mg |
|---|---|---|
| **Nucleus** | Inert beads | 47.00 |
| **FC 1** | Omeprazole<br>Hypromellose<br>Disodium phosphate dihydrate<br>Talc<br>Purified water | 20.00<br>15.28<br>0.50<br>4.78<br>154.75 |
| **FC 2** | Hypromellose<br>Talc<br>Titanium dioxide<br>Purified water | 10.46<br>1.39<br>1.39<br>69.76 |
| **Enteric coat** | Eudragit L30D-55, 30% dry content<br>Triethyl citrate<br>Talc<br>Purified water | 29.97<br>1.35<br>1.35<br>28.89 |
| **Total** | | 112.51 |

**EXAMPLE 6:**

Omeprazole delayed release pellets containing an enteric layer of methacrylic acid copolymer and an intermediate layer. Each film coating (FC) suspension was made and sprayed over the preceding layer in a fluid bed apparatus according with the following components:

| Coat | Component | mg per dose 20mg |
|---|---|---|
| **Nucleus** | Inert beads | 47.00 |
| **FC 1** | Omeprazole<br>Hypromellose<br>Disodium phosphate dihydrate<br>Talc<br>Purified water | 20.00<br>15.28<br>0.50<br>4.78<br>154.75 |
| **FC 2** | Hypromellose<br>Talc<br>Titanium dioxide<br>Purified water | 10.46<br>1.39<br>1.39<br>69.76 |
| **Enteric coat** | Eudragit L30D-55, 30% dry content<br>Triethyl citrate<br>Talc<br>Purified water | 39.71<br>1.79<br>1.79<br>38.28 |
| **Total** | | 116.31 |

**EXAMPLE 7:**

Omeprazole delayed release pellets containing an enteric layer of methacrylic acid copolymer and an intermediate layer. Each film coating (FC) suspension was made and sprayed over the preceding layer in a fluid bed apparatus according with the following components:

| Coat | Component | mg per dose 20mg |
|---|---|---|
| **Nucleus** | Inert beads | 47.00 |
| **FC 1** | Omeprazole<br>Hypromellose<br>Disodium phosphate dihydrate<br>Talc<br>Purified water | 20.00<br>15.28<br>0.50<br>4.78<br>154.75 |
| **FC 2** | Hypromellose<br>Talc<br>Titanium dioxide<br>Purified water | 10.46<br>1.39<br>1.39<br>69.76 |
| **Enteric coat** | Eudragit L30D-55, 30% dry content<br>Triethyl citrate<br>Talc<br>Purified water | 49.95<br>2.26<br>2.26<br>38.28 |
| **Total** | | 120.31 |

**EXAMPLE 8:**

Omeprazole delayed release pellets containing an enteric layer of methacrylic acid copolymer and an intermediate layer. Each film coating (FC) suspension was made and sprayed over the preceding layer in a fluid bed apparatus according with the following components:

| Coat | Component | mg per dose 20mg |
|---|---|---|
| **Nucleus** | Inert beads | 47.00 |

(continued)

| Coat | Component | mg per dose 20mg |
|---|---|---|
| **FC 1** | Omeprazole<br>Hypromellose<br>Disodium phosphate dihydrate<br>Talc<br>Purified water | 20.00<br>15.28<br>0.50<br>4.78<br>154.75 |
| **FC 2** | Hypromellose<br>Talc<br>Titanium dioxide<br>Purified water | 19.73<br>2.63<br>2.63<br>131.63 |
| **Enteric coat** | Eudragit L30D-55, 30% dry content<br>Triethyl citrate<br>Talc | 26.39<br>1.19<br>1.19 |
| | Purified water | 25.44 |
| **Total** | | 122.85 |

**EXAMPLE 9 - STABILITY DATA OF FORMULATION OF EXAMPLE 1 AND 2**

The objective was to measure the stability of the pharmaceutical compositions of the present invention (obtained in the Examples 1 and 2) and compare it with the stability of the oral pharmaceutical compositions of EP 1 086 694 A2.

The purpose of the study is to perform a comparative stability testing of:

- Omeprazole 40 mg pharmaceutical compositions concentrated - Example 1
- Omeprazole 40 mg pharmaceutical compositions concentrated - Example 2
- Omeprazole 40 mg delayed release capsules (EP 1 086 694 A2)

Samples of all the formulations were packaged in 100 ml HDPE bottles and stored for 3 months at 40°C/75%RH. The number of capsules and the quantity of silica gel in the bottle cap for each formulation are described in table 2. Tested parameters were colour of the pellets and total impurities.

Chromatographic conditions: Column: Symmetry C8 15 x 0.46 cm of 5 $\mu$m; Detection: UV 280 nm; Flow: 1.5 ml/min; Column temperature: 40 °C. Mobile phase: organic phase (A) 50% (0.05M $KH_2PO_4$ at pH of 6.5), 50% ACN. Aqueous phase (B) 95% (0.05M $KH_2PO_4$ a pH of 6.5), 5% ACN. Gradient:

| **Time** | **0** | **3** | **25** | **35** | **50** | **60** | **62** | **80** |
|---|---|---|---|---|---|---|---|---|
| **%A** | 0 | 0 | 40 | 40 | 100 | 100 | 0 | 0 |
| **%B** | 100 | 100 | 60 | 60 | 0 | 0 | 100 | 100 |

Solvent (for samples and standard): Prepare a 0.025 M solution of tribasic sodium phosphate. Add 0.5 g of sodium hydroxide per litter and stir to dissolve. Dissolve 1 part of acetonitrile in 3 parts of buffer and mix.

Individual impurities were calculated as follows:

$$\%\text{ individual impurity} = \frac{\text{Area impurity} \cdot \text{Concentration standard} \cdot 100}{\text{Area standard} \cdot \text{Concentration sample}}$$

$$\text{Total impurities} = \Sigma\ \%\text{ individual impurity } (\geq 0.05\%)$$

**Results**

The results of the test under accelerated conditions are summarized in the following table:

| **Drug product** | **Example 1** | **Example 2** | **Omeprazole 40mg delayed release capsules (**EP 1 086 694**)** |
|---|---|---|---|
| **Silica gel in the bottle cap** | **4.2 g** | | |
| **Number of capsules** | **100** | | **28** |
| **g silica per capsule** | **0.042** | | **0.15** |
| Pellets appearance (initial) | Slightly beige | Slightly beige | White to slightly beige |
| Pellets appearance (3 months) | Slightly beige | Slightly beige | Slightly pink |
| Total impurities (3 months) | 0.26 | 0.47 | 0.90 |

## Conclusions

Pharmaceutical compositions of omeprazole obtained in examples 1 and 2 from the present invention are more stable than pharmaceutical composition of omeprazole delayed release capsules of EP 1 086 694 A2. Even with lower amount of silica per capsule, pharmaceutical compositions from examples 1 and 2 have less amount of total number of impurities than the reference formulation. Furthermore, the colour of the pellets in the pharmaceutical compositions of the present invention does not change whereas the colour from the omeprazole capsules from prior-art turns to pink resulting in a partially degradation of the product.

## Claims

**1.** An oral solid pharmaceutical composition comprising pellets which comprise:

a) a core consisting of an inert bead covered with a layer consisting of omeprazole or a pharmaceutically acceptable salt thereof, hydroxypropylmethylcellulose, disodium phosphate dihydrate and talc;
b) at least an intermediate layer; and
c) an enteric coating layer comprising an enteric polymer;

and **characterized in that** the weight percentage of the enteric coating layer is between 20% (w/w) and 40% (w/w) with respect to the total weight of the pellet and wherein the inert bead has a diameter average size lying between about 0.5 mm and 0.9 mm.

**2.** The pharmaceutical composition according to claim 1, wherein the inert bead has a diameter average size lying between about 0.7 mm and 0.9 mm, preferably about 0.8 mm.

**3.** The pharmaceutical composition according to any one of claims 1 to 2, wherein the inert bead is present in an amount lying between about 20% and 50%, preferably between about 20% and 35% or between about 35% and 45%, more preferably about 31% or about 40%, by weight of the pellet.

**4.** The pharmaceutical composition according to any one of claims 1 to 2 wherein the inert bead is present in an amount lying between 20% and 45% by weight of the pellet

**5.** The pharmaceutical composition according to any one of claims 1 to 4, wherein the amount of omeprazole or a pharmaceutically acceptable salt thereof varies from about 10% to 25%, preferably from about 12% to 17% with respect to the weight of the pellet.

**6.** The pharmaceutical composition according to any one of claims 1 to 5, wherein the at least an intermediate layer (b) comprises one layer comprising hydroxypropylmethylcellulose, talc and titanium dioxide.

**7.** The pharmaceutical composition according to any one of claims 1 to 6, wherein the at least an intermediate layer (b) comprises one layer comprising a non-alkaline polymer insoluble in water and optionally a non-alkaline polymer soluble in water.

**8.** The pharmaceutical composition according to claim 7, wherein the at least an intermediate layer (b) comprises one layer comprising ethylcellulose and hydroxypropylmethylcellulose.

**9.** The pharmaceutical composition according to any one of claims 1 to 8, wherein the enteric coating (c) comprises a methacrylate copolymer, a plasticizer and an anti-tacking agent.

**10.** The pharmaceutical composition according to claim 9, wherein the enteric coating (c) comprises methacrylic acid copolymer, triethyl citrate and talc.

**11.** The pharmaceutical composition according to claim 1, wherein said pellets comprises:

a) a core consisting of an inert bead covered with a layer comprising omeprazole or a pharmaceutically acceptable salt thereof, hydroxypropylmethylcellulose, disodium phosphate dihydrate and talc;
b) an intermediate layer comprising hydroxypropylmethylcellulose, talc and titanium dioxide;
c) optionally, another intermediate layer comprising ethylcellulose and hypromellose; and
d) an enteric coating layer comprising methacrylic acid copolymer, triethyl citrate and talc.

**12.** The pharmaceutical composition according to any one of claims 1 to 11 for use in the treatment and/or prophylaxis of ulcers of the stomach and duodenum, and NSAID-induced ulcers; gastroesophageal reflux disease (GERD); severe erosive esophagitis; poorly responsive systematic GERD; pathological hypersecretory conditions such as Zollinger-Ellison Syndrome; and adjunctive treatment of *Helicobacter pylori* infection, alongside antibiotics; or combinations of any of the above disorders.

**Patentansprüche**

**1.** Festes orales Arzneimittel, umfassend Pellets, die umfassen:

a) einen Kern bestehend aus einem inerten Kügelchen, überzogen mit einer Schicht bestehend aus Omeprazol oder einem pharmazeutisch verträglichen Salz davon, Hydroxypropylmethylcellulose, Dinatriumphosphat-Dihydrat und Talk;
b) mindestens eine Zwischenschicht; und
c) eine magensaftresistente Überzugsschicht, die ein magensaftresistentes Polymer umfasst;

und **dadurch gekennzeichnet, dass** das Gewichtsprozent der magensaftresistenten Überzugsschicht zwischen 20% (Gew./Gew.) und 40% (Gew./Gew.) in Bezug auf das Gesamtgewicht des Pellets beträgt und wobei der durchschnittliche Durchmesser des inerten Kügelchen bei zwischen etwa 0,5 mm und 0,9 mm liegt.

**2.** Arzneimittel nach Anspruch 1, wobei der durchschnittliche Durchmesser des inerten Kügelchen bei zwischen etwa 0,7 mm und 0,9 mm, vorzugsweise bei etwa 0,8 mm liegt.

**3.** Arzneimittel nach einem der Ansprüche 1 bis 2, wobei das inerte Kügelchen in einer Menge zwischen etwa 20% und 50%, vorzugsweise zwischen etwa 20% und 35% oder zwischen etwa 35% und 45%, mehr bevorzugt etwa 31% oder etwa 40% in Bezug auf das Gewicht des Pellet vorliegt.

**4.** Arzneimittel nach einem der Ansprüche 1 bis 2, wobei das inerte Kügelchen in einer Menge zwischen etwa 20% und 45% in Bezug auf das Gewicht des Pellet vorliegt.

**5.** Arzneimittel nach einem der Ansprüche 1 bis 4, wobei die Menge an Omeprazol oder eines pharmazeutisch verträglichen Salzes davon von etwa 10% bis 25%, vorzugsweise von etwa 12% bis 17% in Bezug auf das Gewicht des Pellets variiert.

**6.** Arzneimittel nach einem der Ansprüche 1 bis 5, wobei die mindestens eine Zwischenschicht (b) eine Schicht umfasst, die Hydroxyproplymethylcellulose, Talk und Titandioxid umfasst.

7. Arzneimittel nach einem der Ansprüche 1 bis 6, wobei die mindestens eine Zwischenschicht (b) eine Schicht umfasst, die ein nicht-alkalisches wasserunlösliches Polymer und gegebenenfalls ein nicht-alkalisches wasserlösliches Polymer umfasst.

8. Arzneimittel nach Anspruch 7, wobei die mindestens eine Zwischenschicht (b) eine Schicht umfasst, die Ethylcellulose und Hydroxypropylmethylcellulose umfasst.

9. Arzneimittel nach einem der Ansprüche 1 bis 8, wobei der magensaftresistente Überzug (c) ein Methacrylat-Copolymer, einen Weichmacher und ein anti-Haft-Agens umfasst.

10. Arzneimittel nach Anspruch 9, wobei der magensaftresistente Überzug (c) ein Methacrylsäure-Copolymer, Triethylcitrat und Talk umfasst.

11. Arzneimittel nach Anspruch 1, wobei die Pellets umfassen:

a) einen Kern bestehend aus einem inerten Kügelchen, überzogen mit einer Schicht umfassend Omeprazol oder ein pharmazeutisch verträgliches Salz davon, Hydroxypropylmethylcellulose, Dinatriumphosphat-Dihydrat und Talk;
b) einer Zwischenschicht, die Hydroxypropylmethylcellulose, Talk und Titandioxid umfasst;
c) gegebenenfalls eine weitere Zwischenschicht, die Ethylcellulose und Hypromellose umfasst; und
d) eine magensaftresistente Überzugsschicht, die Methacrylsäure-Copolymer, Triethylcitrat und Talk umfasst.

12. Arzneimittel nach einem der Ansprüche 1 bis 11 zur Verwendung bei der Behandlung und/oder Vorbeugung von Magen- und ZwölffingerdarmGeschwüren, und von NSAR-induzierten Geschwüren; gastroösophagealer Reflux-Krankheit (GERD); schwerer erosiver Ösophagitis; schlecht ansprechender systematischer GERD; pathologischen hypersekretorischen Zuständen wie Zollinger-Ellison-Syndrom; und bei begleitender Behandlung von *Helicobacter pylori*-Infektion zusammen mit Antibiotika; oder Kombinationen von beliebigen der obigen Erkrankungen.

## Revendications

1. Composition pharmaceutique solide orale comprenant des granulés qui comprennent :

a) un noyau constitué par une bille inerte enrobée d'une couche constituée par de l'oméprazole ou un sel pharmaceutiquement acceptable de celui-ci, de l'hydroxypropylméthylcellulose, du phosphate disodique dihydraté et du talc ;
b) au moins une couche intermédiaire ; et
c) une couche d'enrobage entérique comprenant un polymère entérique ;

et **caractérisée en ce que** le pourcentage en poids de la couche d'enrobage entérique est compris entre 20 % (m/m) et 40 % (m/m) par rapport au poids total du granulé et **en ce que** la bille inerte a un diamètre moyen compris entre environ 0,5 mm et 0,9 mm.

2. Composition pharmaceutique selon la revendication 1, dans laquelle la bille inerte a un diamètre moyen compris entre environ 0,7 mm et 0,9 mm, de préférence d'environ 0,8 mm.

3. Composition pharmaceutique selon l'une quelconque des revendications 1 à 2, dans laquelle la bille inerte est présente en une quantité comprise entre environ 20 % et 50 %, de préférence entre environ 20 % et 35 % ou entre environ 35 % et 45 %, plus préférablement d'environ 31 % ou d'environ 40 %, en poids du granulé.

4. Composition pharmaceutique selon l'une quelconque des revendications 1 à 2 dans laquelle la bille inerte est présente en une quantité comprise entre 20 % et 45 % en poids du granulé.

5. Composition pharmaceutique selon l'une quelconque des revendications 1 à 4, dans laquelle la quantité d'oméprazole ou d'un sel pharmaceutiquement acceptable de celui-ci varie d'environ 10 % à 25 %, de préférence d'environ 12 % à 17 % par rapport au poids du granulé.

6. Composition pharmaceutique selon l'une quelconque des revendications 1 à 5, dans laquelle l'au moins une couche

intermédiaire (b) comprend une couche comprenant de l'hydroxypropylméthylcellulose, du talc et du dioxyde de titane.

**7.** Composition pharmaceutique selon l'une quelconque des revendications 1 à 6, dans laquelle l'au moins une couche intermédiaire (b) comprend une couche comprenant un polymère non alcalin insoluble dans l'eau et facultativement un polymère non alcalin soluble dans l'eau.

**8.** Composition pharmaceutique selon la revendication 7, dans laquelle l'au moins une couche intermédiaire (b) comprend une couche comprenant de l'éthylcellulose et de l'hydroxypropylméthylcellulose.

**9.** Composition pharmaceutique selon l'une quelconque des revendications 1 à 8, dans laquelle le revêtement entérique (c) comprend un copolymère de méthacrylate, un plastifiant et un agent anti-adhérent.

**10.** Composition pharmaceutique selon la revendication 9, dans laquelle le revêtement entérique (c) comprend un copolymère d'acide méthacrylique, du citrate de triéthyle et du talc.

**11.** Composition pharmaceutique selon la revendication 1, dans laquelle lesdits granulés comprennent :

a) un noyau constitué par une bille inerte enrobée d'une couche comprenant de l'oméprazole ou un sel pharmaceutiquement acceptable de celui-ci, de l'hydroxypropylméthylcellulose, du phosphate disodique dihydraté et du talc ;
b) une couche intermédiaire comprenant de l'hydroxypropylméthylcellulose, du talc et du dioxyde de titane ;
c) facultativement une autre couche intermédiaire comprenant de l'éthylcellulose et de l'hypromellose ; et
d) une couche d'enrobage entérique comprenant un copolymère d'acide méthacrylique, du citrate de triéthyle et du talc.

**12.** Composition pharmaceutique selon l'une quelconque des revendications 1 à 11, destinée à être utilisée dans le traitement et/ou la prophylaxie d'ulcères de l'estomac et du duodénum, et les ulcères induits par des NSAID ; la maladie de reflux gastro-oesophagien (RGO) ; l'oesophagite érosive sévère ; un RGO systématique répondant mal aux traitements ; les pathologies hypersécrétrices telles que le syndrome de Zollinger-Ellison ; et un traitement en complément d'antibiotiques pour traiter une infection par *Helicobacter pylori*; ou des combinaisons de l'un quelconque des troubles ci-dessus.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1086694 A2
- WO 2011054930 A
- WO 9601623 A
- EP 1552833 A
- WO 2012092486 A
- EP 1108425 A
- US 5385739 A
- WO 2004016242 A
- WO 2004096218 A
- EP 1086694 A